# EUROPEAN PATENT APPLICATION

(11) **EP 2 272 515 A1**
(43) Date of publication of application: **12.01.2011**
(21) Application number: 09719846.9
(22) Date of filing: 06.03.2009
(51) Int. Cl.: A61K 31/47, A61K 31/06, A61K 31/192, A61K 38/16, A61K 38/43, A61K 45/06, A61P 25/28

(54) **NOVEL USES OF SODIUM 4-PHENYLBUTYRATE (4 PBA) AND THE PHARMACEUTICALLY ACCEPTABLE SALTS THEREOF**

(30) Priority: 13.03.2008 ES 200800736
(71) Applicant: Proyecto de Biomedicina Cima, S.L., 31008 Pamplona - Navarra (ES)
(72) Inventor: FRECHILLA MANSO, Diana Sara, E-31008 Pamplona (Navarra) (ES); GARCÍA OSTA, Ana Maria, E-31008 Pamplona (Navarra) (ES); PÉREZ MEDIAVILLA, Luis Alberto, E-31008 Pamplona (Navarra) (ES); RICOBARAZA ABARQUERO, Ana Lourdes, E-31008 Pamplona (Navarra) (ES)
(74) Representative: Ungria Lopez, Javier
(86) International application number: PCT/ES2009/000121
(87) International publication number: WO 2009/112609

(57) **Abstract**

Novel uses for 4-phenylbutyrate (4PBA) and Its pharmaceutically acceptable salts

The use of 4PBA in the manufacture of a medicament useful to treat cognitive disorders in dementias coursing with tauopathies is described.

The treatment with 4PBA improves cognitive deficit in a classic murine experimental model of Alzheimer's Disease (AD) (Tg 2576) that is accompanied by decreasing levels of a marker characteristic to AD pathologies, such as phosphorylated Tau, and increasing levels in the synthesis of some synaptic plasticity marker proteins such as GluR1 and PSD95. As mediating mechanism for the therapeutic effect we propose a decreasing stress in the endoplasmic reticulum as manifested by increasing levels of the GRP78 protein. On the other hand its inhibiting effect on histone deacetylation and its involvement in chromatin remodeling facilitates protein synthesis processes, which improves synaptic plasticity.

## Description

### TECHNICAL FIELD OF THE INVENTION

The present invention belongs to the field of biotechnology applied to the medical-pharmaceutical area for the treatment of cognitive disorders in dementias coursing with tauopathies, and specially Alzheimer's disease.

More specifically, the present invention provides a novel treatment for those conditions based on the use of 4PBA.

### STATE OF THE ART PRIOR TO THE INVENTION

Alzheimer's disease (AD) is the most common neurodegenerative disease and the first cause of dementia (Cummings and Cole, 2002). The physiopathological characteristics of Alzheimer's disease (AD) are related to aggregated protein deposits: intracellular phosphorylated tau protein aggregates in neurofibrillary tangles and extracellular aggregates of β-amyloid (Aβ) in senile plaques. Therefore, Alzheimer's diseases is a very representative example of diseases related to abnormal protein folding (Selkoe, 2004).

The endoplasmic reticulum (ER) is a site that plays a particularly significant role in the quality control process of protein-related processes, since it regulates the synthesis, folding and circulation of proteins in the body. Continuous protein production requires maintaining a strict quality control process, which takes place in the ER. The ER does not always function with appropriate accuracy and badly folded proteins may accumulate in its interior. This situation may occur because some proteins are difficult to fold, such as those that are responsible for causing Alzheimer's diseases, and also because proteins are synthesized at a faster speed than that at which the ER can fold them. This causes a "stress" situation for the ER. When this situation is reached, sensors are activated and start sending a series of signals intended to stop synthesis of the greater part of the proteins, and also to regulate folding speed, such that it is possible to correctly fold all proteins and ultimately help the ER recover from the stressful situation.

ER stress is a key event that triggers and mediates neuronal death in Alzheimer's disease. Several types of cellular stress may cause accumulation of abnormally folded proteins in the ER's lumen, this stress situation triggering the activation of the protein quality control mechanisms in order to prevent the toxic actions of such accumulation (or the so called "Unfolded Protein Response" or UPR). Activation the UPR control mechanism produces a general reduction in protein synthesis, greater protein degradation, and an increase in the transcription of genes that encode molecular chaperons that reside In the ER, such as the glucose-regulated proteins (GRPs) GRP78/ Bip and GRP94, together with the protein disulfide isomerase (PDI), in order to facilitate the protein folding process (Kozutsumi et al., 1998). As such, Katayama et al., (1999) demonstrated that there were less ER-residing molecular chaperons, such as GRP78 and GRP94, in the brains of patients suffering from Alzheimer's disease when compared to the quantity found in brains of individuals of the same age from the control group, a situation that may lead the endoplasmic reticulum to vulnerability when under stress. These researchers suggested that the expression of GRP78 acts as a protection against ER stress-caused neuronal death. Consequently, increasing GRP78 expression may allow developing therapeutic strategies to combat Alzheimer's disease (AD).

If the UPR mechanism is not capable of resolving ER stress experienced by neuronal cells, it is very possible that the disease progresses, triggering neuron apoptosis. This would be the situation for the most common and devastating neurodegenerative diseases, including Alzheimer's, Parkinson's and Huntington's diseases, all characterized by the accumulation and aggregation of unfolded proteins. Therefore it is not surprising that many researchers have found evidence that the UPR mechanism Is also activated in these extreme conditions. In this case, however, activation of this mechanism ends up causing more harm than benefit since the initial response is protective but the final response is destructive.

There are several situations that favor the accumulation of proteins in the brain, Aging represents the greatest risk factor for developing AD. The capacity for inducing chaperon molecule expression before a situation of cellular stress diminishes with age. It has also been demonstrated that the UPR mechanism is not activated correctly in elderly animals. Thus, age increases the ER's susceptibility to stress and to the toxic effect of protein accumulation. Endoplasmic reticulum stress may be induced by hypoxia and/or hypoglycemia episodes, both being common situations during the normal aging process. Also, if proteosome activity decreases with age, the body is more susceptible to formation of abnormal protein aggregates.

With age, a scenario may be generated In the brain in which neurodegenerative diseases related to abnormal protein accumulation have a high probability of developing.

Interrupting the ER protein system is a factor that has been included in several human diseases besides Alzheimer's, Parkinson's and Huntington's diseases (Katayama et al., 2001; Nishitoh et al., 2002; Ryu et al., 2002). There is a series of compounds called "chemical chaperons" such as glycerol, dimethylsulfoxide (DMSO) and sodium 4-phenylbutyrate or 4PBA that have shown the improvement of abnormal processes during protein folding and localization (Sato et al., 1996; Chaudhuri and Paul, 2006; Kubota et al., 2006), and whose administration has inhibited neuronal death In Parkinson's disease murine models (Masatoshi I, et al., 2007).

4PBA is a low molecular weight fatty acid that has been approved for use in the clinical practice as ammonia sequestering agent in children suffering from urea cycle disorders (Maestri et al., 1996) and in the treatment of sickle cell anemia and thalassemia due to its capacity to activate fetal hemoglobin transcription (Dover et al., 1994; Collins et al., 1995). Also documented is its beneficial effect in patients with Spinal Muscular Atrophy (Mercuri E., et al., 2004; Mercuri E., et al., 2007). It has been shown that 4PBA can act as chemical chaperon reverting abnormal protein localization and/or aggregation of proteins associated to human diseases (Rubenstein and Zeitlin, 2000; Kubota et al., 2006). Also, 4PBA can play a protective role in certain diseases associated to neuroinflammatory response, such as multiple sclerosis and ischemia (Dasgupta et al., 2003; Qi et al., 2004). Medical literature also cites its beneficial effects on polyglutaminic toxicity (Steffan et al., 2001).

Potentially beneficial for treating AD and other diseases that have an inflammatory component, patent WO99/ 026657 protects the use of compounds inhibiting the inducible nitric oxide synthase (iNOS) . Reference to PBA is marginally mentioned in the context of a list of iNOS-inhibiting compounds (lovastatin, forskolin, mevastatine, etc.), yet the description section does not contain any experiment directed to show the beneficial effect of PBA in an AD contrasted model.

It has also been shown that in the signaling effects common to situations of overloaded or stressed endoplasmic reticulum, aberrant tau phosphorylation plays a decisive role, which is shared by many degenerative diseases (Hernández and Avila, 2007). Phosphorylated tau is free, has a reduced affinity for microtubules and therefore, little capacity to promote their assembly. The agent responsible for this tau aberrant phosphorylation is the over-activated glycogen synthase kinase 3α/β (GSK) (Kim et al., 2007). Phospho-tau aggregates are one of the identifying characteristics of AD and other tauopathies. The strategies in place for cellular protection against ER overload can directly inhibit GSK3 activity and therefore reduce tau phosphorylation and its toxicity It is possible that tau phosphorylation is an integrated component of the neural machinery, to the extent that the lack of regulation of said function leads to memory deterioration in the development of diseases such as Alzheimer's (Arendt et al., 2003; Ikeda et al., 2007).

Another reaction related to learning processes behavior is histone acetylation. Histone acetylation mediates the mechanisms that regulate gene transcription by means of chromatin modification, and therefore it has been recently involved in synaptic plasticity. It has been verified that administering 4PBA increases histone acetylation in a murine model expressing Amyotrophic Lateral Sclerosis (Petri S, et al., 2006). Consequently, it was speculated that 4PBA, by inhibiting histone deacetylase may induce a transcriptional modification that may lead to activation of neuronal plasticity genes. Andreassi et al (2004) related 4PBA administration directly to the increase in the complete expression of genes whose loss is responsible for Spinal Muscular Atrophy.

It is then probable that increasing H4 histone acetylation may regulate transcription programs that are relatively specific. AMPA receptor trafficking through the post-synaptic membrane is an indication of rapid transmission and of synaptic plasticity.

Genetic transcription requires that the activation of transcription factors, but it also requires induction of dynamic changes in the organization of the chromatin that conducts genetic expression. Histone hyperacetylation releases histones from their bond to chromatin, with subsequent effects on genetic transcription processes (Lea and Randolph, 1998) and on protein synthesis. It has been shown that RNA synthesis and "de novo" protein synthesis are necessary in the process of production of long term memory in various vertebrate and invertebrate animal species and also they will probably mediate the formation of new synaptic connections (Tully et al., 2003). It is believed that long term memory formation entails long-lasting effects in genetic expression, and there exists more and more evidence that histone modifications and DNA methylation do probably participate in this process (Bailey et al., 2004; Levenson and Sweatt, 2005). The work of Fischer et al., (2007) showed that increasing histone acetylation by injecting a histone deacetylase inhibitor (HDAC) or by enriching the environment; It facilitated memory function in an experimental model in which a neurodegenerative disease was provoked in mice. The improvement in learning processes and memory consolidation were correlated to increased transcription and more directly with increased regulation of synaptic plasticity markers and dendritic markers, which suggests a new mechanism that may improve memory function.

The histone acetylation process controls the transcription of genes that are necessary for memory consolidation and long term synaptic potentiation (LTP) (Bailey et al., 2004; Levenson and Sweatt, 2005; Fischer et al., 2007). The aberrant activity of histone acetyltransferase (HAT) and of histone deacetylase (HDAC) may also be a mechanism common to neurological diseases that contributes to neurodegeneration, such as strokes, Huntington's disease, amyotrophic lateral sclerosis, Friedreich's ataxia, and Alzheimer's disease (according to the revision published by Langley et al in 2005). Some studies have shown that genetic transcription is deregulated in some brain regions in association to the progression of the disease, and support the notion of the existence of an RNA transcription disruption mechanism in some degenerative disorders (Anderson et al., 2007).

The GluR1 subunit in the AMPA receptor and PSD95 are key postsynaptic markers in synaptic formation and function. PSD95 and GluR1 postsynaptic deficits found in the brains of patients suffering from Alzheimer's disease and in the brains of Tg2576 transgenic mice may contribute to synaptic dysfunction and to deficits of the cognitive function (Almeida et al., 2005). Based on this notion, researchers observed that the expression-reduction markers for the GluR1 subunit at the AMPA receptor and PSD95 In the hippocampus of Tg2576 transgenic mice treated with 4PBA did show improvement of cognitive functions. Several studies have already profiled the spectrum of 4PBA-altered genes that prevent neurotoxicity in various experimental models (Chang and Min, 2002; Kang et al., 2002; Ryu et al., 2005).

The physiopathology of many degenerative disorders, such as Alzheimer's disease, and also neuroprotection probably comprise numerous mechanisms. In the particular case of Alzheimer's disease, these pathogenic mechanisms include mainly Aβ aggregation and deposition with plaque development, tau hyperphosphorylation with tangle formation, neurovascular dysfunction, and other mechanisms such as cell-cycle abnormalities, inflammatory processes, oxidative stress, and mitochondrial dysfunction. The central hypothesis for the cause of Alzheimer's disease is the amyloid cascade hypothesis.

It is calculated that in the next 50 years the number of persons affected by AD will triplicate and reach 13.2 millions in 2050. This increase is due to the continuous growth of elder population. Each four years life expectancy is increased by one year, for Alzheimer's sufferers also. This means that those affected by this disease will live longer (currently the life expectancy rate for these patients is between 10 and 12 years after diagnosis). In terms of preventative treatment the vaccine research was terminated due to the side effects on the central nervous systems.

These considerations show that Alzheimer's disease will be the great challenge for sociosanitary planning in the next 50 years.

Since there is not yet an effective treatment for Alzheimer's disease, this invention describes the capacity of 4PBA, by means of its multiple mechanisms, to revert the deterioration of the learning process and the memory function that are typical of Alzheimer's disease in a murine model.

Kim et al 2004 demonstrate that the expression of amyloid precursor protein C-terminal fragments (APP-CTs) induce increases in acetylation of histone 3 and histone 4 and that the cytotoxicity induced by APP-CTs are significantly increased by the treatment with sodium butyrate in NGF-differentiated PC12 cells and rat primary cortical neurons. Taken together, the results suggest that APP-CTs exert neurotoxicity by transcription dependent mechanisms and this might contribute to the pathogenesis of AD.

Fischer et al 2007 show that sodium butyrate, not 4PBA, restores histone acetylation status and learning and memory In a mouse model of neurodegeneration, i.e. the bi-transgenic CK-p25 Tg mouse, in which expression of p25, a protein implicated in various neurodegenerative diseases, is under the control of the CamKII promoter and can be switched on or off with a doxycicline diet. However, the animal model used by Fischer et al. 2007 is not a representative model for Alzheimer's disease, since it does not reproduce the pathogenic mechanisms around the human amyloid-beta protein or aberrant tau phosphorylation. In particular it only encompasses impaired expression of the protein p25, a truncated form of p35, which is a regulatory subunit of cyclindependent kinase-5 (CDK5).

In contrast, the inventors of the present invention, by using a validated and consensus transgenic mouse model for Alzheimer's disease, i.e. Tg2576 mice, which expresses a human amyloid-beta precursor protein (APP) variant linked to Alzheimer's disease, have surprisingly found that sodium 4-phenylbutyrate is effective in the treatment of Alzheimer's disease. The inventors have been able to show that clinical symptoms such as memory acquisition and retention are improved and importantly, have also demonstrated that tau hyperphosphorylation levels are diminished after administration of 4PBA.

### DESCRIPTION OF THE INVENTION

The present invention describes the potentially beneficial role of 4PBA in Alzheimer's disease type amnesia. We found that treating 16 months old Tg2576 transgenic mice with 4PBA for 5 weeks did revert the spatial memory deficit. The results obtained suggest that it is probable that the effect of 4PBA is mediated, on the one hand, by the protection it confers to cells against the effects of endoplasmic reticulum stress by increasing the levels of molecular chaperons and inducing tau dephosphorylation, and on the other hand by increasing acetylation of the H4 histone, which in turns induces the expression of synaptic markers, which in turn lead to recovery of long term spatial memory.

It was also found that the GRP78 levels are lower in the hippocampus of Tg2576 transgenic mice; even more, it was observed that GRP78 expression reached similar levels in non-transgenic mice after treatment with 4PBA, which is in agreement with Katayama's observations (1999).

It was also observed that a reduction in H4 acetylation in the cerebral cortex of Tg2576 transgenic mice could be related to a densely packed chromatin structure, which would correspond to transcriptional repression.

The results of the investigation suggest the hypothesis that 4PBA, because it increases histone H4 acetylation, it also activates transcription and the synthesis of proteins that favor neuronal plasticity (AMPA receptor GluR1 subunit and PSD95) and of endoplasmic reticulum proteins (GRP78), which in turn entail the correct function of the hippocampus and lead to recovering deteriorated memory function.

It was also observed an increase in the level of tau phosphorylation at the Ser202/Thr205 (AT8) location in the hippocampi of Tg2576 transgenic mice treated with saline solution while the tau phosphorylation levels in the transgenic mice treated with 4PBA and those of non-transgenie mice did not differ. The present invention demonstrates that reducing the levels of phospho-tau improves cognitive functions in transgenic mice. This finding is in agreement with recent observations suggesting that tau reduction has beneficial effects in preventing behavioral deficits in transgenic mice expressing a protein precursor of human amyloid without altering their Aβ levels (Roberson et al., 2007).

Because one of the mechanisms of action of 4PBA is the decrease of the hyperphosphorylated form of tau, it can be assumed that it will also be effective for the cognitive treatment of other dementias coursing with tauopathy besides Alzheimer's disease, such as frontotemporal dementia, progressive supranuclear paralysis (PSP), Pick's disease and Lewy body dementia.

The present invention relates to 4PBA or a pharmaceutical acceptable salt thereof for use in the prevention and/or treatment of Alzheimer's disease. In other words, the present invention relates to the use of 4PBA or a pharmaceutical acceptable salt thereof for the manufacture of a medicament for the prevention and/or treatment of Alzheimer's disease. Similarly, the present invention provides a method for preventing or treating Alzheimer's disease that comprises administering to a subject in need of said prevention or treatment a pharmaceutically effective amount of 4PBA or a pharmaceutical acceptable salt thereof.

The term "4PBA" as used herein also comprises those metabolites or compounds that are the result of its metabolization in the body. The embodiments of the present invention relating to the different therapeutical uses of this compound also comprise the use of its metabolite, i.e. phenylacetate.

In one embodiment, the present invention refers to 4PBA or any one of its pharmaceutically acceptable salts, or to a composition comprising 4PBA or any one of its pharmaceutically acceptable salts, for use in the prevention or treatment of dementias coursing with tauopathy, and more particularly, for its use in the prevention or treatment of cognitive disorders in said dementias.

The invention also refers to the use of 4PBA or any one of its pharmaceutically acceptable salts, or to a composition comprising 4PBA or any one of its pharmaceutically acceptable salts for the manufacture of a medicament for the prevention or treatment of dementias coursing with tauopathy, and in particular for cognitive disorders associated with said dementias.

In one embodiment of the invention refers particularly to the 4-PBA sodium salt to be used in the prevention or treatment of cognitive disorders associated to dementias coursing with tauopathy.

4PBA is 4-phenylbutyric acid (4-phenylbutyrate), a commercially available product that can be obtained from companies such as SIGMA-ALDRICH (Product N°: P21005); having as registry CAS number: 1821-12-1; its chemical formula is C₆H₅ (CH₂)₃COOH.

4PBA sodium salt is also a commercially available product that can be obtained, for instance, from BIOMOL International L.P. (Palatine House, Matford Court, Exeter EX2 8NL, UK; Catalogue No.: EI320); registry CAS number 1716-12-7; its chemical formula is C₆H₅(CH₂)₃COONa; and its structural formula is:

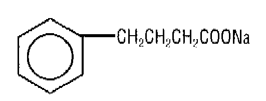

Alternatively, this compound can also be obtained using known methods.

Buphenyl® and Ammonaps® are trademarks for sodium phenylbutyrate compositions, authorized by FDA and EMEA, respectively, for the treatment of urea cycle disorders. It can also be found as TriButyrate® (Triple Crown America).

The term "pharmaceutically acceptable" means that the compound or combination of compounds must be compatible with the remaining ingredients of a formulation, and that is not deleterious for the patient or subject receiving it. For therapeutic use, salts of 4PBA are those wherein the counter-ion is pharmaceutically acceptable.

In one embodiment of the invention the composition that comprises 4PBA or one of its pharmaceutically acceptable salts is a pharmaceutical composition that also comprises a vehicle or pharmaceutically acceptable excipient.

The pharmaceutically acceptable salts as mentioned hereinabove are meant to comprise the therapeutically active non-toxic salt forms which 4PBA is able to form. The pharmaceutically acceptable salts can conveniently be obtained by treating the acid form of 4PBA with such appropriate cations. Appropriate basic salts comprise those formed with organic cations such as benzathine, chloroprocaine, choline, diethanolamine, ethylenediamine, meglumine, procaine; and those formed with metallic cations such as aluminum, calcium, lithium, magnesium, potassium, sodium, and zinc.

Conversely said salt forms can be converted by treatment with an appropriate acid into the free form.

The term salts is also meant to include the hydrates or solvates that 4PBA is able to form, including, e.g. the alcoholates such as methanolates or ethanolates. The term "solvate" refers to those crystal forms of 4PBA that coomprise either stoichiometric or non-stoichiometric amounts of solvent. Since water is a solvent, solvates also include hydrates. The term "pseudopolymorph" is synonym to solvate since it applies to polymorphic crystalline forms that have solvent molecules incorporated in their lattice structures. Examples of solvates are hydrates and alcoholates such as methanolates or ethanolates.

The term "prevention" refers to the act of "preventing", understood as avoiding the onset, existence or, alternatively, as delaying the onset or recurrence of a disease, disorder or condition to which the term is applied to, or of one or several of the symptoms associated to the disease, disorder or condition.

The term "treatment" refers to the act of "treating", a term that is also understood as reverting, relieving or inhibiting the progression of the disease, disorder or condition to which the term is applied to, or of one or several of the symptoms associated to the disease, disorder or condition.

"Dementias coursing with tauopathy" refers to neurodegenerative diseases in which there is an alteration in the tau protein metabolism and in which one of the most relevant symptoms or consequences is the onset of cognitive disorders. Particularly, the use of 4PBA or its salts is useful for the prevention or treatment of cognitive disorders associated to such dementias.

One particular embodiment of the invention relates to the prevention or treatment of diseases such as Alzheimer's disease, frontotemporal dementia, progressive supranuclear paralysis, corticobasal degeneration, Pick's disease, Lewy body dementia, argyrophillic grain dementia, type C Niemann-Pick disease, or pugilistic dementia.

A more particular embodiment of the invention relates to the prevention or treatment of Alzheimer's disease, and particularly of the cognitive disorders associated to this disease.

A more preferred embodiment of the present invention is the use 4PBA sodium salt in the manufacture of a medicament for the treatment of the previously described diseases.

Another aspect of the invention refers to a composition comprising 4PBA or any one of its pharmaceutically acceptable salts for use in the prevention or treatment of dementias coursing with tauopathy, and particularly cognitive disorders associated to these diseases. In a particular embodiment the composition comprises 4PBA sodium salt. A particular embodiment concerns a pharmaceutical composition comprising 4PBA sodium salt and a pharmaceutically acceptable carrier for use in the prevention and/or treatment of Alzheimer's disease.

Previous studies have already shown that improving the learning process and the memory function is not always associated to detectable alterations of Aβ levels in the brain (Dodart et al., 2002; Gong et al., 2004). If this information is considered together with the information presented in the present invention, the results suggest that the relation between soluble and insoluble concentrations of Aβ in the brain and memory deterioration observed in transgenic mice is a relation specific to the task and also complex. Additionally, a clear correlation in extensive studies has not been found between the deposited forms of Aβ (such as in the case of plaques) and memory deterioration in patients affected by Alzheimer's disease (Hyman et al., 1984). In fact, more recent reports suggest that the levels of soluble Aβ may be more correlated to neurodegeneration and memory deterioration (Lue et al., 1999; McLean et al., 1999; Lesné et al., 2007).

Therefore, another embodiment of the present invention, intended to potentiate its curative effect, is a combination comprising 4PBA and/or any one of its pharmaceutically acceptable salts as first active ingredient together with a second active ingredient agent that is an agent to induce or facilitate β-amyloid deposits clearing in the brain. A more preferred embodiment uses as clearing agent of said deposits, metal chelating agents, catabolizing enzymes present in the soluble form of the β-amyloid peptide, cerebrolysine or nitrophenols.

Another embodiment of the present invention provides a pharmaceutical combination comprising 4PBA or a pharmaceutically acceptable salt thereof as a first active ingredient, a second active ingredient agent that is an inducer or facilitator agent for clearing β-amyloid deposits in the brain, and a pharmaceutically acceptable carrier. A more preferred embodiment uses as clearing agent of said deposits, metal chelating agents, catabolizing enzymes present in the soluble form of the β-amyloid peptide, cerebrolysine or nitrophenols.

An even more preferred embodiment is the use of clioquinol, cerebrolysine, or 2,4-dinitrophenol or 3-nitrophenol as deposit-clearing inducing agent.

An even more preferred embodiment is the use of clioquinol, cerebrolysine, IDE insulin degrading enzyme or neprilisin, or 2,4-dinitrophenol or 3-nitrophenol as deposit-clearing inducing agent.

Another embodiment of the invention is therefore the use of a product containing 4PBA and/or any one of its pharmaceutically acceptable salts as first active ingredient, and a second active ingredient that is an inducing or facilitating agent of the clearing of brain β-amyloid deposits, as a combined preparation for simultaneous use for the prevention and/or treatment of cognitive disorders in dementias coursing with tauopathy. Another embodiment features the same combined preparation designed for the separate use of both active principles, and a further embodiment is designed for its sequential use.

As such, the two components or active ingredients in the pharmaceutical combination of the invention may be part of the same unitary pharmaceutical formulation; or part of separate pharmaceutical formulations for the coadministration of the two components or active ingredients as part of the same therapeutic regime. When the two components are administered in separate formulations, it is not necessary for them to be administered at the same time, although it could be done in this manner if so desired.

In an additional aspect the invention refers to a product or kit comprising a pharmaceutical combination comprising 4PBA and/or any one of its pharmaceutically acceptable salts together with an inducing or facilitating agent for clearing β-amyloid deposits in the brain, in any one of the stipulated embodiments herein.

In another aspect, the invention refers to said pharmaceutical combination or kit comprising said pharmaceutical combination for its use in the prevention or treatment of dementias coursing with tauopathy, and particularly of the associated cognitive disorders. Said combination or kit are useful for the manufacture of a medicament for the prevention of said dementias, particularly any of those previously specified, and more particularly Alzheimer's disease.

Other aspect of the invention also refers to a method for the prevention or treatment of dementias coursing with tauopathy, and particularly the associated cognitive disorders, which comprises administering to a subject in need of said prevention and/or treatment a pharmaceutically effective quantity of 4PBA or any one of its pharmaceutically acceptable salts, or a composition comprising of 4PBA and/or any one of its pharmaceutically acceptable salts, or a pharmaceutical combination comprising of 4PBA and/or any one of its pharmaceutically acceptable salts together with an inducing or facilitating agent for clearing the β-amyloid deposits formed in the brain.

The term "pharmaceutically effective amount" refers to a quantity of compound or combination of active compounds that is effective in treating the disease, that improves, attenuates or eliminates one or several of its symptoms; or that prevents or delays the onset of one of more symptoms of the disease.

The dose of the active substances of the present invention, i.e. 4PBA and/or pharmaceutically acceptable salts thereof, to be administered depends on the individual case and, as customary, is to be adapted to the conditions of the individual case for an optimum effect. Thus it depends, of course, on the frequency of administration and on the potency and duration of action of the compound employed in each case for therapy or prophylaxis, but also on the nature and severity of the disease and symptoms, and on the sex, age, weight, co-medication and individual responsiveness of the human or animal to be treated and on whether the therapy is acute or prophylactic.

The therapeutically effective amount of 4PBA and/or pharmaceutically acceptable salts thereof, may be in the range from 1 mg to 200 g per day, from 2 mg to 150 g per day, from 5 mg to 100 g per day, from 10 mg to 75 g per day, from 20 mg to 75 g per day, from 50 mg to 75 g per day, from 0.1 g to 75 g per day, from 0.2 g to 75 g per day, from 0.5 g to 75 g per day, from 1 g to 50 g per day, from 5 g to 50 g per day.

The term "subject" refers to an animal, particularly mammals like primates, dogs, cats, bovine animals, horses, sheep, and humans. It is particularly applied to human mammals of both genders.

A preferred mode of administration of the manufactured medicament containing 4PBA for the uses described above is in the form of tablets containing its sodium salt. The pharmaceutical preparation of these tablets may comprise excipients such as microcrystalline cellulose, magnesium stearate or colloidal silicium dioxide.

Another preferred form of administration is as powder containing the 4PBA sodium salt and an excipient that allows it to be water soluble. The pharmaceutical preparation of this powder may comprise excipients such as calcium stearate or colloidal silicium dioxide.

### BRIEF DESCRIPTION OF THE FIGURES

**Figure 1A****:** Latency times experienced by the transgenic mice treated with 4PBA and saline solution compared to those of non-transgenic mice at different days when levels were measured with a Morris test during the visible platform phase.
**Figure 1B****:** Latency times experienced by the transgenic mice treated with 4PBA and saline solution compared to those of non-transgenic mice at different days when levels were measured with a Morris test during the invisible platform phase.
**Figure 1C****:** Time the transgenic mice treated with 4PBA and saline solution remained in the correct quadrant as compared to those of non-transgenic mice at different days when levels were measured during a Morris test.
**Figure 2A****:** Aβ42 and Aβ40 peptide-derived levels found in an extract of the brain cortex of transgenic mice treated with 4PBA and saline solution.
**Figure 2B****:** Marking scheme of plaques formed by Aβ peptide accumulation in the brain of transgenic mice treated with 4PBA and saline solution as compared to those of non-transgenic mice.
**Figure 3A****:** Relation between phosphorylated tau levels versus normal protein in transgenic mice treated with 4PBA and saline solution as compared to those of non-transgenic mice.
**Figure 3B****:** Relation between phosphorylated GSK3 (inactive form) levels versus unphosphorylated protein (active form) in transgenic mice treated with 4PBA and saline solution as compared to those of non-transgenic mice.
**Figure 4****:** Levels of GRP78 expression in hippocampi extracts of transgenic mice treated with 4PBA and saline solution as compared to those of non-transgenic mice.
**Figure 5****:** Acetylated histone 4 (AcH4) and histone 3 (AcH3) levels in cortex extracts of transgenic mice treated with 4PBA and saline solution as compared to those of non-transgenic mice.
**Figure 6****:** Levels of expression of the GluR1, PSD95 and MAP-2 proteins in membrane enriched protein hippocampi extracts obtained from transgenic mice treated with 4PBA and saline solution as compared to those of non-transgenic mice.

### EMBODIMENTS OF THE INVENTION

The present invention is additionally illustrated by the following Examples that, together with the Figures described previously illustrate the experimental methodology used for its development. It is understood that the skilled in the art will be able to understand the modifications, variations and changes that can be implemented within the scope of the present invention.

### EXAMPLES

### Example 1. Murine animal experimentation model and treatment thereof

We used Tg2576 transgenic mice with Alzheimer's Disease expressing the 695-aa human APP isoform which contains the double APPswe Swedish mutation [(APP695) Lys670 → Asn, Met671 → Leu] triggered by a cryonic hamster promoter. In the Tg2576 transgenic mice suffering from Alzheimer's disease used in the experimental murine model, the Aβ peptide content in the brain accumulates exponentially between their 7^{th} and 12^{th} months of life. The mice thus manipulated showed deterioration of the memory function when they were subjected to the Morris aquatic maze test when they reached the age between 12 and 15 months. Therefore 16 months old Tg2576 transgenic mice females were treated once a day intraperitoneally with 200 mg/kg 4PBA or its vehicle during 5 weeks. To do this a 4PBA solution was prepared titrating equimolecular amounts of 4-phenylbutyric acid (Sigma) and sodium hydroxide at a pH: 7.4. A group of normal mice, not genetically altered, from same similar strains and ages was used as control group. All the clinical test procedures were performed according to the European and the Spanish legislation (86/ 609/CEE; RD 1201/2005).

### Example 2. Morris water maze (MWM)

The Morris water maze was used to evaluate the operational and reference memory functions in Tg2576 transgenic mice treated with 4 PBA as described previously in the literature (Ribe et al., 2005). Groups of Tg2576 female mice treated with 4PBA (n = 6), vehicle (n = 7), and non-transgenic litter siblings (n = 10) were subjected to spatial learning and memory tests using the Morris water maze when they became 16 months old. The maze consisted of a circular pool filled with water at 20° C. The mice received prior training in a pool with a visible platform during 3 consecutive days (8 tests per day) being able to swim towards a platform raised above the water level. The training with hidden platform took 9 consecutive days (4 tests per day) during which all mice were allowed 60 seconds to find the platform submerged 1cm below the water surface. Those mice that could not find the platform were guided to it. All mice were allowed a 20 second rest period on the platform and then they were lifted from it and taken back to their cages. At the beginning of the 4^{th}, 7^{th} and last days of the test, a prior test was performed during which the platform was removed from the pool and the mice were allowed to look for it during 60 seconds. All the trials were monitored with a VHS camera and the Watermaze software to analyze escape latencies and the percentage of time of occupation of each pool quadrant during prior tests (using the Ethovision software, Wageninge, Netherlands). Those mice that did not learn to locate the visible platform or mice that exhibited abnormal swimming patterns or floated persistently were excluded from the analyses.

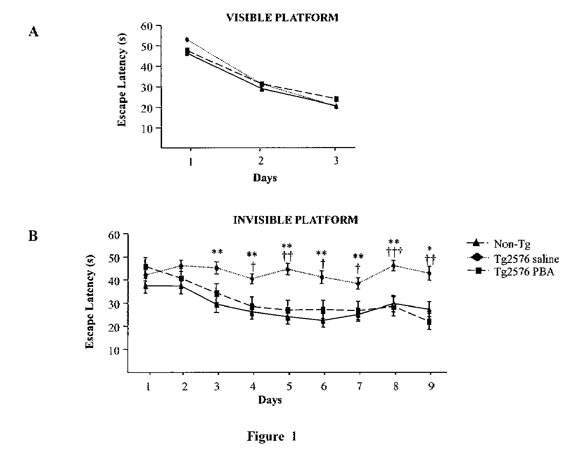

In order to study the effects of 4PBA on the cognitive function it was analyzed whether 5 weeks of 4PBA treatment would alleviate learning deficits exhibited by the 16 month old Tg2576 transgenic mice during the Morris water maze trial. At the end of the treatment, proficiency showed by the 16 month old transgenic mice treated with vehicle was lower than the proficiency showed by non-genetically altered mice of same age. Conversely, the proficiency showed by transgenic mice treated with 4PBA did not differ from that shown by mice in the control group of same age (Figure 1B).

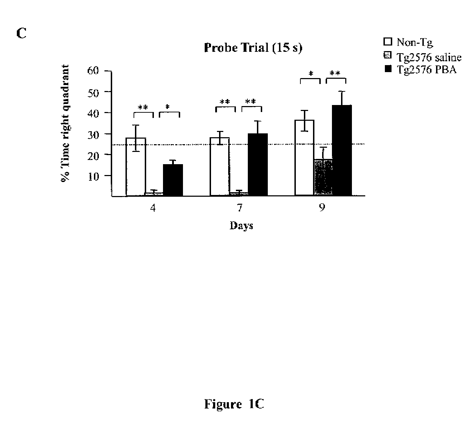

After 12, 24 and 32 tests, all mice were subjected to a prior test during which they were put to swim In the pool from which the platform had been removed for a period of 15 seconds. A memory retention measurement is the percentage of 15 seconds of swimming in the quadrant were the platform used to be during the training sessions (the target quadrant). The percentage of time the transgenic mice treated with vehicle spent in the target quadrant was significantly lower than the time the mice from the control group spent in said quadrant. The amount of time the 4 PBA treated transgenic mice spent in said quadrant did not differ from the amount of time the mice from the control group of same age spent in said quadrant (Figure 1C). Although the differences became evident in the invisible platform test, the escape latencies did not differ significantly during the first training tests in which the visible platform was used (Figure 1A). These data suggest that chronic administration of 4PBA improves the memory function in Tg2576 transgenic mice relative to the water maze test.

### Example 3. Method to determine Aβ levels

Since 4PBA treatment showed to clearly benefit the memory acquisition and retention process, the next aspect was to explore the effect of the Aβ levels and tau pathology in Tg2576 transgenic Mice. Mice were sacrificed 24 hours after performing the last test in the Morris water maze. The cortex and hippocampus were sectioned and used to perform biochemical analyses. Then the levels of Aβ40 and Aβ42 in the cerebral cortex were analyzed by a sandwich type ELISA test.

The cerebral cortexes were homogenized in a buffer containing 5 M guanidine HCl and 50 mM Tris HCl at a pH = 8, protease inhibitors (Roche's CompleteTM Protease Inhibitor Cocktail), and phosphatase inhibitors (0.1 mM Na₃VO₄, 1 mM NaF). The homogeneates thus obtained were sonicated during 2 min and spun at 100,000 x g during 1 h. Aliquots of the supernatant were freeze-dried at -80° C and the protein concentration determined using the Bradford method with the Bio-Rad kit. Aβ42 levels were measured with an extra sensitive sandwich-type ELISA kit from Biosource (Camarillo, Ca, USA). To do this 300 µg of total supernatant protein were deposited directly onto the ELISA plates in duplicate following manufacturer's instructions.

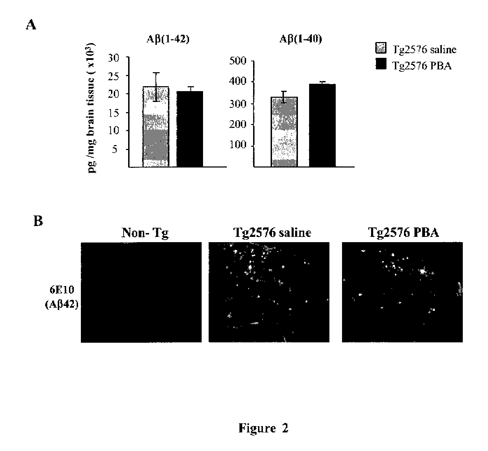

As can be seen in Fig. 2A, no difference was observed in the Aβ40 or Aβ42 levels in Tg2576 transgenic mice treated with vehicle as compared to transgenic mice treated with 4PBA. Aβ was not detected in non-transgenic litter siblings. Similar results were obtained when the Aβ levels and the percentage of the Aβ immunoreactivity area were measured in order to calculate the plaque load in the hippocampus (Figure 2B).

### Example 4. Production of protein extracts and Western Blot type immunological analysis

Mice were sacrificed by cervical dislocation and their hippocampi were sectioned quickly from the brain. Total homogeneates were obtained by homogenizing the hippocampus tissue in an ice-cooled RIPA buffer solution (50 mM Tris-HCL, pH = 7.4, 0.25% DOC, 1% Nonidet P-40, 150 mM NaCl, 1 mM EDTA, 1 mM PMSF, 1 µg/ml leupeptin, 1 µg/ml aprotinin, 1 mM Na₃VO₄, 1 mM NaF) spun at 14,000 x g, 4° C for 20 min and the aliquots obtained from the supernatant were then freeze-dried at -80° C. A standard method was used to obtain the membrane enriched protein fraction (P2 membrane protein), The hippocampus tissue was homogenized in an ice-cooled Tris-EDTA buffer solution (10 mM Tris-HCl and 5 mM EDTA, pH = 7.4) containing 320 mM of sucrose and the previously described protease and phosphatase inhibitors. The homogenized tissue was spun at 700 x g for 10 min. The resulting supernatant was spun again at 37,000 x g during 40 min at 4° C. Finally, the pellet (P2) was resuspended in 10 mM of Tris-HCl buffer solution (pH = 7.4) containing the enzyme inhibitor mix described above. Protein concentration was determined in both cases (using the Bradford test from Bio-Rad) and the aliquots stored at - 80° C until further use. For the Western blot type analysis, the P2 membrane fractions were solubilized in denaturating conditions adding a 0.1 volume of 10% sodium deoxycholate in a 500 mM Tris-HCl buffer solution (pH = 9). The samples were incubated during 30 min at 36° C and diluted by adding a 0.1 volume of 500 mM Tris-HCl (pH = 9) / 1% Triton X-100. After centrifuging the samples at 37,000 x g during 10 min at 4° C the resulting supernatant was stored at -80° C.

To analyze the histones, extracts from the mice's frontal cerebral cortex were obtained and homogenized in a cold lysis buffer medium with protease inhibitors (0.2 M NaCl, 0.1 M HEPES, 10% glycerol, 200 mM NaF. 2 mM Na₄P₂O₇, 5 mM EDTA, 1 mM EGTA, 2 mM DTT, 0.5 mM PMSF, 1 mM Na₃VO₄, 1 mM benzamydine, 10 µg/ml leupeptin, 400 U/ml aprotinin) and then centrifuged at 14.000 x g at 4° C during 20 min. The aliquots obtained from the supernatant were stored at -80° C. Total protein concentration was obtained with the Bradford test from Bio-Rad Bradford (BioRad laboratories, Hercules, California).

To carry out the Western Blot analyses, protein samples were mixed in buffers having the same volumes (2 x Laemmli) resolved in SDS-polyacrylamide gels and transferred to a nitrocellulose membrane. The membranes were then blocked in 5% milk, 0.05% Tween-20 in PBS or TBS blocking solution followed by an incubation period during the entire night with the following antibodies: mouse monoclonal anti-phospho tau AT8 (Pierce), rabbit polyclonal anti-pGSK3-Ser9 (Cell Signalling), rabbit polyclonal anti-GSK3, goat polyclonal anti-GRP78 (Santa Cruz), rabbit polyclonal anti-histone acetylate 4 and 3 (Upstate), rabbit polyclonal anti-GluR1 (Chemicon), mice monoclonal anti-PSD95 (Chemicon), rabbit polyclonal anti-MAP2 (Chemicon, Temecula, CA) mouse monoclonal anti-actin, and mouse monoclonal anti-tubulin (Sigma) in the corresponding buffer solutions. All the antibodies were used at 1: 1000 dilution except the mouse monoclonal anti-actin and mouse monoclonal anti-tubulin that were used at 1:10000. After two washings in PBS/Tween-20 or TBS/Tween20 and one washing in PBS or TBS, immunolabelled protein bands were detected using an anti-rabbit or anti-mouse HRP conjugate (Santa Cruz, 1:5000 dilution) followed by a chemiluminescence system (ECL Amersham Biosciences), and autoradiographic exposure to HyperfilmTMECL (Amersham Biosciences). The signals were quantified using the Scion Image program (Scion Corporation).

### Example 5. Pathological markers of Alzheimer's disease

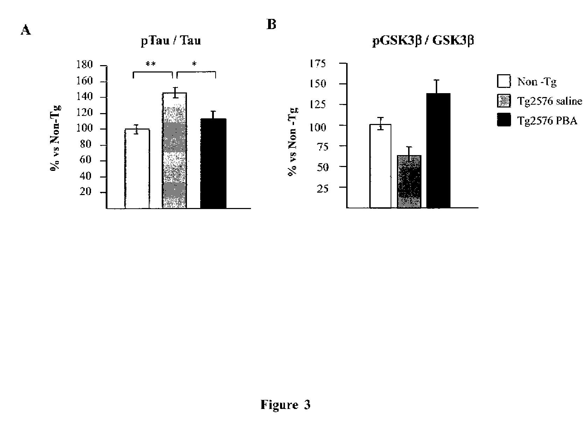

In order to investigate the changes that could explain the differences observed in the learning capacity of the transgenic mice after treatment, the levels of tau phosphorylation were analyzed in the hippocampi extracted from the mice using AT8, a phosphospecific antibody capable of recognizing aberrant hyperphosphorylated epitopes in Ser-202/Thr-205. It was found that tau phosphorylation increased in the hippocampi from the 16 month old Tg2576 transgenic mice as compared with those of non-transgenic mice, while the global levels of tau were not altered. An interesting finding was that no differences were found in the phosphorylated tau at the AT8 site of transgenic mice treated with 4PBA when compared with the same function in non-transgenic mice (Fig. 3A).

The tau phosphorylation function is regulated by several kinase and phosphatase proteins. It has been shown that GSK3b participates in pathological tau phosphorylation. Levels of inactive GSK3b phosphorylated at Ser 9 were measured, and it was observed that said levels were higher in the hippocampi of 4PBA treated transgenic mice when compared with those treated with the vehicle, which would explain the decrease in the phosphorylated tau form observed in treated mice (Figure 3B).

### Example 6. Endoplasmic reticulum stress markers

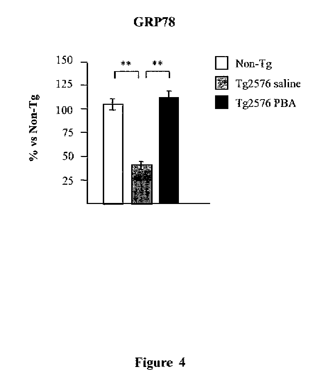

The next step of the study was to analyze the levels of the molecular chaperon residing in the endoplasmic reticulum GRP78, in the hippocampus of transgenic mice and in that of the litter siblings from the control group. It was found that the GRP78 levels were lower in the hippocampus of 16 months old transgenic mice when compared with those of mice of same age. For comparison, the GRP78 levels of 4PBA treated transgenic mice did not differ from the levels of GRP78 found in non-transgenic mice, which suggests that the effect of the 4PBA treatment may be due in part to an increase in the expression levels of GRP78 (Fig. 4).

### Example 7. Synaptic plasticity markers

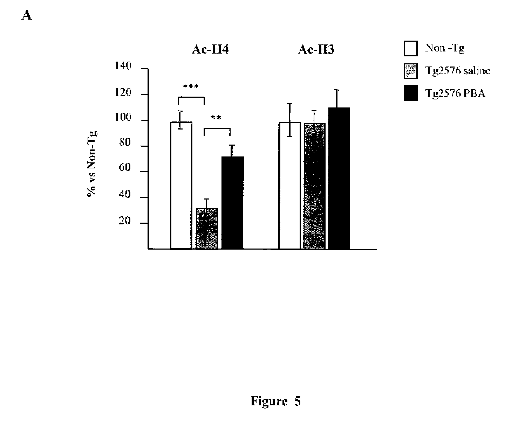

16 month old Tg2576 transgenic mice showed very low levels of H4 acetylation when compared with those of the same aged mice from the control group. Therefore, induction of acetylation of the cortical histone 4 (H4) was found in transgenic mice that had been treated with 4PBA (Fig. 5).

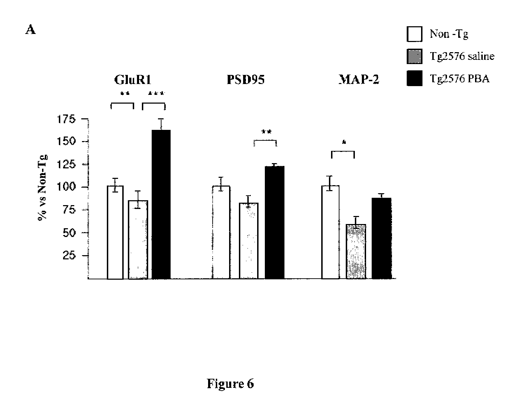

AMPA receptor GluR1 subunit, PSD95, and MAP-2 are plasticity markers with crucial importance in synapse formation and function. Postsynaptic deficits of PSD95, GluR1, and MAP-2 described in brains from AD patients and Tg2576 mice may contribute to synaptic dysfunction with resultant impaired learning. The next step was to investigate whether the expression of these synaptic markers levels was modified after treatment with 4PBA, and a robust increase (161.37 ± 27.5%) was found in the expression levels of the GluR1 subunit of the AMPA receptor in membrane enriched protein extracts from the hippocampi of the group of transgenic mice that had been treated with 4PBA when compared to the levels of expression found in mice treated with the vehicle (81.18 ± 9.5%), and compared against the levels found in the group of non-genetically altered mice (100.0 ± 6.5%). 4PBA treatment also induced significantly the expression of PSD95 levels in the same extracts (Fig.6).

The microtubule-associated protein-2 (MAP-2) is a protein localized primarily in neuronal dendrites. MAP-2 expression coincides with dendritic outgrowth, branching, and post-lesion dendritic remodeling, suggesting that this protein plays a crucial role in plasticity (Johnson and Jope, 1992). We performed quantitative western blot analysis to measure MAP-2, and we found that MAP-2 expression levels are significantly decreased in Tg2576 mice (54.9±10.7) compared with non-transgenic mice (100.0±14.9) and it is partially restored after 4PBA treatment (77.7±11.6) (Fig.6).

### BIBLIOGRAPHY

■ Almeida CG, Tampellini D, Takahashi RH, Greengard P, Lin MT, Snyder EM, Gouras GK (2005). Beta-amyloid accumulation in APP mutant neurons reduces PSD-95 and GluR1 in synapses. Neurobiol Dis 20:187-198.
■ Anderson AN, Roncaroli F, Hodges A, Deprez M, Turkheimer FE (2007) Chromosomal profiles of gene expression in Huntington's disease. Brain.
■ Andreasi C, Angelozzi C, Tiziano FD, Vitali T, De Vicenzi E, Boninsegna A, et al., (2004). Phenylbutyrate increases SMN expression in vitro: relevance for treatment of spinal muscular atrophy. Neuromuscul Disord 14 (2) :130 -135
■ Arendt T, Stieler J, Strijkstra AM, Hut RA, Rudiger J, Van der Zee EA, Harkany T, Holzer M, Hartig W (2003). Reversible paired helical filament-like phosphorylation of tau is an adaptive process associated with neuronal plasticity in hibernating animals, J. Neurosci., 23:6972 - 6981.
■ Bailey CH, Kandel ER, Si K (2004). The persistence of long-term memory: a molecular approach to self-sustaining changes in learning-induced synaptic growth. Neuron 44: 49 - 57.
■ Cummings JL, Cole G (2002). Alzheimer disease. JAMA 287: 2335 - 2338.
■ Chang KT, Min KT. (2002). Regulation of lifespan by histone deacetylase. Ageing Res Rev 1: 313 - 326.
■ Chaudhuri TK, Paul S (2006). Protein-misfolding diseases and chaperone-based therapeutic approaches. FEBS J 273: 1331 - 1349.
■ Collins AF, Pearson HA, Giardina P, McDonagh KT, Brusilow Sw, Dover GJ (1995). Oral sodium phenylbutyrate therapy in homozygous beta thalassemia: a clinical trial. Blood 85: 43 - 49.
■ Dasgupta S, Zhou Y, Jana M, Banik NL, Pahan K (2003). Sodium phenylacetate inhibits adoptive transfer of experimental allergic encephalomyelitis in SJL/J mice at multiple steps. J Immunol 170: 3874 - 3882.
■ Dodart JC, Bales KR, Gannon KS, Greene SJ, DeMattos RB, Mathis C, DeLong CA, Wu S, Wu X, Holtzman DM, Paul SM. (2002) Immunization reverses memory deficits without reducing brain Abeta burden in Alzheimer's disease model. Nat Neurosci 5: 452 - 457.
■ Dover GJ, Brusilow S, Charache S (1994). Induction of fetal hemoglobin production in subjects with sickle cell anemia by oral sodium phenylbutyrate. Blood 84(1): 339 - 343.
■ Fischer A. Sananbenesi F, Wang X, Dobbin M, Tsai LH (2007). Recovery of learning and memory is associated with chromatin remodelling. Nature 447, 178 - 182.
■ Gong B, Vitolo 0V, Trinchese F, Liu S, Shelanski M, Arancio 0 (2004). Persistent improvement in synaptic and cognitive functions in an Alzheimer mouse model after rolipram treatment. J Clin Invest 114; 1624 - 1634.
■ Hernández F, Ávila J (2007). Tauopathies. Cell Mol Life Sci 64: 2219 - 2233.
■ Hyman BT, Van Hoesen GW, Damasio AR, Barnes CL (1984). Alzheimer's disease: cell-specific pathology isolates the hippocampal formation Science 225: 1168 - 1170.
■ Ikeda Y, Ishiguro K, Fujita SC (2007). Ether stress-induced Alzheimer-like tau phosphorylation in the normal mouse brain. FEBS Lett 581: 891-897.
■ Kang HL, Benzer S, Min KT (2002). Life extension in Drosophila by feeding a drug. Proc Natl Acad Sci USA 99: 838 - 843.
■ Katayama T, Imaizumi K, Sato N, Miyoshi K, Kudo T, Hitomi J, Morihara T, Yoneda T, Gomi F, Mori Y, Nakano Y, Takeda J, Tsuda T, Itoyama Y, Murayama 0, Takashima A, St George-Hyslop P, Takeda M, Tohyyama M, (1999). Presenilin-1 mutations downregulate the signalling pathway of the unfolded-protein response, Nat Cell Biol 1, 479-485.
■ Katayama T, Imaizumi K, Honda A, Yoneda T, Kudo T, Takeda M, Mori K, Rozmahel R, Fraser P, George-Hyslop PS, Tohyama M (2001). Disturbed activation of endoplasmic reticulum stress transducers by familial Alzheimer's disease-linked presenilin-1 mutations. J Biol Chem 276: 43446 - 43454.
■ Katayama T, Imaizumi K, Manabe T, Hitomi J, Kudo T, Tohyama M (2004). Induction of neuronal death by ER stress in Alzheimer's disease. J Chem Neuroanat 28: 67 - 78.
■ Kim HS, Kim EM, Kim NJ, Chang KA, Choi Y, Ahn KW, Lee JH, Kim S, Park CH, Suh YH (2004). Inhibition of histone deacetylation enhances the neurotoxicity induced by the C-terminal fragments of amyloid precursor protein. J Neurosci Res 75:117 - 124
■ Kim HJ, Rowe M, Ren M, Hong JS, Chen PS, Chuang DM (2007). Histone deacetylase inhibitors exhibit anti-inflammatory and neuroprotective effects in a rat permanent ischemic model of stroke: multiple mechanisms of action. J Pharmacol Exp Ther 321: 892-901.
■ Kozutsumi Y, Segal M, Normington K, Gething MJ, Sambrook J (1998). The presence of malfolded proteins in the endoplasmic reticulum signals the induction of glucose-regulated proteins. Nature 332:462-464.
■ Kubota K, Niinuma Y, Kaneko M, Okuma Y, Sugai M, Omura T, Uesugi M, Uehara T, Hosoi T, Nomura Y (2006). Suppressive effects of 4-phenylbutyrate on the aggregation of Pael receptors and endoplasmic reticulum stress. J Neurochem 97:1259-1268.
■ Langley B, Gensert JM, Beal MF, Ratan RR Curr Drug Targets CNS (2005). Remodeling chromatin and stress resistance in the central nervous system: histone deacetylase inhibitors as novel and broadly effective neuroprotective agents. Neurol Disord 4:41 - 50.
■ Lea MA, Randolph VM (1998). Induction of reporter gene expression by inhibitors of histone deacetylase. Anticancer Res 18: 2717-2722.
■ Lesné S, Kotilinck L, Ashe KH (2007). Plaque-bearing mice with reduced levels of oligomeric amyloid-beta assemblies have intact memory function. Neuroscience Epub ahead of print.
■ Levenson JM, Sweatt JD (2005) Epigenetic mechanisms in memory formation. Nat Rev Neurosci 6: 108- 118.
■ Lue LF, Kuo YM, Roher AE, Brachova L, Shen Y, Sue L, Beach T, Kurth JH, Rydel RE, Rogers J (1999). Soluble amyloid beta peptide concentration as a predictor of synaptic change in Alzheimer's disease Am J Patho 155: 853-862.
■ Maestri NE, Brusilow SW, Clissold DB, Bassett SS (1996). Long-term treatment of girls with ornithine transcarbamylase deficiency. N Engl J Med 335:855 - 859.
■ Masatoshi I, Yoshihisa K, Hiroki T, Takashi Y, Kazuyuki T, Yuka K, Takashi T, Kanji Y, Masahiko K, Yasunobu 0, Takahiro T, Hiroyoshi A, Shun S (2007). Neurodegeneration of mouse nigrostriatal dopaminergic system induced by repeated oral administration of rotenone is prevented by 4-phenylbutyrate, a chemical chaperone, Jour of Neurochem 101: 1491-1504.
■ McLean CA, Cherny RA, Fraser FW, Fuller SJ, Smith MJ, Beyreuther K, Bush Al, Masters CL (1999). Soluble pool of Abeta amyloid as a determinant of severity of neurodegeneration in Alzheimer's disease. Ann Neurol 46: 860-866.
■ Mercuri E, Bertini E, Messina S, Pelliccioni M, D' Amico A, Colitto F, et al., (2004). Pilot trial of phenylbutryrate in spinal muscular atrophy. Neuromuscul Disord 14 (2): 130 - 135.
■ Mercuri E, Bertini E, Messina S, Solari A, D'Amico A, Angelozzi C, et al., (2007). Randomized, double-bind, placebo-controlled trial of phenylbutyrate in spinal muscular atrophy. Neurol 68 (1): 51- 55.
■ Nishitoh H, Matsuzawa A, Tobiume K, Saegusa K, Takeda K, Inoue K, Hori S, Kakizuka A, Ichijo H (2002). ASK1 is essential for endoplasmic reticulum stress-induced neuronal cell death triggered by expanded polyglutamine repeats. Genes Dev 16: 1345 -55.
■ Petri s, Kiaci M, Kipani K, Chen J, Calingasan NY, Crow JP, et al., (2006). Additive neuroprotective effects of a histone deacetylase inhibitor and a catalytic antioxidant in a transgenic mouse model of amyotrophic lateral sclerosis. Neurol Dis 22 (1): 40-49.
■ Qi X, Hosoi T, Okuma Y, Kaneko M, Nomura Y (2004). Sodium 4-phenylbutyrate protects against cerebral ischemic injury. Mol Pharmacol 66: 899-8908.
■ Ribé EM, Pérez M, Puig B, Gich Y, Lim F, Cuadrado M, Sesma T, Catena S, Sánchez B, Nieto M, Gómez-Ramos P, Morán MA, Cabodevilla F, Samaranch L, Ortiz L, Pérez A, Ferrer I, Avila J, G6mez-Isla T (2005). Accelerated amyloid deposition, neurofibrillary degeneration and neuronal loss in double mutant APP/tau transgenic mice. Neurobiol Dis 20: 814-822.
■ Roberson ED, Scearce-Levie K, Palop JJ, Yan F, Cheng IH, Wu T, Gerstein H, Yu GQ, Mucke L (2007). Reducing endogenous tau ameliorate amyloid beta-induced deficits in an Alzheimer's disease mouse model. Science 316:750-754.
■ Rubenstein RC, Zeitlin PL (2000). Sodium 4-phenylbutyrate downregulates Hsc70: Implications for intracellular trafficking of DeltaF508-CFTR. Am J Physiol Cell Physiol 278: 259 - 267.
■ Ryu EJ, Harding HP, Angelastro JM, Vitolo OV, Ron D, Greene L (2002). Endoplasmic reticulum stress and the unfolded protein response in cellular models of Parkinson's disease. J Neurosci 22: 10690 - 10698.
■ Ryu H, Smith K, Camelo SI, Carreras I, Lee J, Iglesias AH, Dangond F, Cormier KA, Cudkowicz ME, Brown RH Jr, Ferrante RJ (2005). Sodium phenylbutyrate prolongs survival and regulates expression of anti-apoptotic genes in transgenic amyotrophic lateral sclerosis mice. J Neurochem 93: 1087- 1098.
■ Sato S, Ward CL, Krouse ME, Winc JJ, Kopito RR (1996) Glycerol reverses the misfolding phenotype of the most common cystic fibrosis mutation. J Biol Chem 271:635-638.
■ Selkoe DJ(2004). Cell biology of protein misfolding: the examples of Alzheimer's and Parkinson's diseases, Nat Cell Biol 6: 1054-1061.
■ Steffan JS, Bodai L, Pallos J, Poelman M, McCampbell A, Apostol BL, Kazantsev A, Schmidt E, Zhu YZ, Greenwald M, Kurokawa R, Housman DE, Jackson GR, Marsh JL, Thompson LM (2001). Histone deacetylase inhibitors arrest polyglutamine-dependent neurodegeneration in Drosophila. Nature 413:739-743.
■ Tully T, Bourtchouladze A, Scott R, Tallman J (2003). Targeting the CREB pathway for memory enhancers, Nat Rev Drug Discov 2: 267-277.
■ WO/1999/026657. Inderjit, S. (1999). Inhibitors of nitric oxide synthase.

## Claims

1. 4-phenyl butyrate (4PBA) or a pharmaceutical acceptable salt thereof for use in the prevention and/or treatment of Alzheimer's disease.

2. Use of 4PBA or a pharmaceutical acceptable salt thereof for the manufacture of a medicament for the prevention and/or treatment of Alzheimer's disease.

3. The 4PBA according to any one of claims 1-2, wherein the pharmaceutical acceptable salt thereof is 4PBA sodium salt.

4. A pharmaceutical composition comprising 4PBA sodium salt and a pharmaceutically acceptable carrier for use in the prevention and/or treatment of Alzheimer's disease.

5. A pharmaceutical combination **characterized in that** it comprises 4PBA or any one of its pharmaceutically acceptable salts as first active ingredient, a second active ingredient that is an inducer or facilitator agent for clearing brain β-amyloid deposits, and a pharmaceutically acceptable carrier.

6. The combination according to claim 5 wherein it comprises 4PBA sodium salt as first ingredient.

7. The combination according to claims 5 or 6 wherein the brain β-amyloid deposits clearing inducing agent is a metal chelating agent.

8. The combination according to claim 7 wherein said metal chelating agent is clioquinol.

9. The combination according to claims 5 or 6 wherein the brain β-amyloid deposits clearing inducing agent is a catabolizing enzyme.

10. The combination according to claim 9 wherein said inducing agent is the IDE insulin degrading enzyme or neprilisin.

11. The combination according to claims 9 or 10 wherein the brain β-amyloid deposits clearing inducing agent is cerebrolysin.

12. The combination according to claims 9 or 10 wherein the brain β-amyloid deposits clearing inducing agents are nitrophenols.

13. The combination according to claim 12 wherein said nitrophenol is 2,4 - dinitrophenol or 3-nitrophenol.

14. The pharmaceutical combination according to any one of claims 5-13 for use in the prevention or treatment of Alzheimer's disease.

15. A method for preventing or treating Alzheimer's disease that comprises administering to a subject in need of said prevention or treatment a pharmaceutically effective amount of 4PBA or a pharmaceutically acceptable salt thereof or a pharmaceutical combination according to any one of claims 5-13.
